# EUROPEAN PATENT APPLICATION

(11) **EP 4 818 319 A1**
(43) Date of publication of application: **07.10.2026**
(21) Application number: 24896642.6
(22) Date of filing: 28.11.2024
(51) Int. Cl.: C12N 15/11, C12N 15/12, C12N 15/10, C12N 15/67

(54) **3'UTR FOR IMPROVING TRANSLATION PERFORMANCE OF RNA MOLECULE**

(30) Priority: 30.11.2023 CN 202311628997
(71) Applicant: Themedium Therapeutics Co., Ltd., Suzhou, Jiangsu 215011 (CN)
(72) Inventor: ZHANG, Wenjing, Suzhou, Jiangsu 215011 (CN); MO, Yicheng, Suzhou, Jiangsu 215011 (CN); KANG, Tao, Suzhou, Jiangsu 215011 (CN); ZHANG, Jingming, Suzhou, Jiangsu 215011 (CN)
(74) Representative: V.O.
(86) International application number: PCT/CN2024/135241
(87) International publication number: WO 2025/113556

(57) **Abstract**

The present application relates to an artificial nucleic acid molecule, related products thereof, and its use in the preparation of drugs for DNA- or RNA-based therapies. It further related to the use of a 3' untranslated region (3'-UTR) element in said artificial nucleic acid molecule to facilitate the translation of the coding region of the nucleic acid molecule into a polypeptide or protein encoded by said coding region, as well as methods for obtaining RNA, polypeptides, or proteins. By incorporating a specific 3'-UTR element capable of enhancing RNA translation performance, the artificial nucleic acid molecule of the present application exhibits superior RNA translation performance, thereby demonstrating significant potential and promising application prospects in the development of RNA-based therapeutics.

## Description

### CROSS-REFERENCE

The present application claims priority to Chinese Patent Application No. 202311628997.0 filed on Nov. 30, 2023 and titled "3'UTR for improving the translation performance of RNA molecules", which is incorporated herein by reference in its entirety.

### TECHNICAL FIELD

The present application relates generally to the field of molecule biology and bioengineering technology. Specifically, it relates to an artificial nucleic acid molecule, a vector comprising same, a host cell comprising the vector, a pharmaceutical composition and a kit comprising the aforementioned, and the use of the aforementioned substances in the preparation of medicines for DNA or RNA-based therapies. It also relates to the use of 3'-untranslated region element (3'-UTR) contained in said artificial nucleic acid for translating coding region of the artificial nucleic acid molecule into a polypeptide or a protein encoded by said coding region, as well as methods for obtaining RNA, polypeptides, or proteins.

### BACKGROUND

In recent years, RNA vaccines have emerged as a highly attractive type of vaccine. Compared to traditional inactivated virus vaccines, viral vector vaccines, and recombination protein vaccines, RNA vaccines offer advantages such as rapid research and development, good safety, high efficacy, ease of standardized production, short production cycles, and low costs. Furthermore, unlike DNA, RNA does not have the risk of integrating into the host cell genome. Given these advantages, RNA not only holds significant potential in vaccine development but also shows great promise in other therapeutic areas, including protein supplementation, antibody therapy, gene editing, cell therapy, and cytokine therapy.

The operational framework of RNA-based therapeutics revolves around the intracellular delivery of exogenously transcribed RNA molecules and the exogenous RNA molecules leverage the host cell's endogenous translation machinery to encode therapeutic proteins. Enhancing translation efficiency and duration of RNA within cells can increase the expression level and retention time of the therapeutic protein, thereby reducing dosage, decreasing administration frequency, and improving therapeutic efficacy. Such advancements can provide broader application prospects for RNA-based therapies.

Optimizing various elements of RNA, including the 5' cap, 5' untranslated region (UTR), coding sequence (CDS) of target gene, 3' UTR, polyadenylated tail (polyA), and nucleotide modifications, has been reported to enhance the translational performance of RNA. Among these, UTRs are recognition regions of cellular machinery related to RNA translation, degradation or stability, controlling RNA translation performance by regulating both translation efficiency and stability. The lengths of UTRs typically span from tens to hundreds of nucleotides. Given this range, it is reasonable to expect that the sequences and structures of UTRs exhibit extensive variability. This variability offers significant potential for modulating RNA translation performance. Consequently, the screening and design of appropriate UTRs to enhance RNA translation performance have become one of the key focused fields.

### SUMMARY

Currently, UTR research strategies mainly include the screening and modification of natural UTRs, as well as computer-aided UTR design. Typically, the UTRs used for screening and modification are derived from natural UTRs of cellular or viral mRNAs. However, previous studies have demonstrated that high-performing UTR sequences not always derive from the UTR regions of natural mRNAs, and effective internal ribosome entry site (IRES) sequences can also arise outside the IRES regions of mRNAs. This implies that concentrating solely on the UTR regions of mRNAs might cause us to overlook numerous opportunities to identify novel and high-performing UTR sequences.

Therefore, the present application utilizes total RNA from various types of cells to establish a library of sequences with length of tens to hundreds of nucleotides to serve as a 3'UTR library. Using cells as the screening environment, the duration of RNA in cells is gradually extended to enrich stable RNA. Subsequently, the 3' UTR elements stably enriched in the cells are sequenced. The translation performance of RNAs with these individual 3' UTR elements, as well as their extended longer versions, is subsequently assessed. These RNAs encode different reporter proteins (via distinct CDS regions). And the contexts for assessing translation performance include various cell lines in vivo models. The overarching goal of this process is to identify universal 3'UTR sequence elements that have the potential to enhance RNA translation performance.

By using the method mentioned above, the inventors have successfully screened and obtained 23 sequence elements that can serve as 3'UTRs, as shown in Table 1. As indicated in Table 1, these sequence elements not only come from 3'UTR regions of natural genes but also originate from CDS, 5'UTR, 5'UTR+CDS, or 3'UTR+CDS regions of natural genes. Additionally, not only using individual 3'UTR elements can enhance the translation performance of RNA molecules, the inventors have discovered that employing a tandem arrangement of several identical or different 3'UTR elements can also enhance the translation performance of RNA molecules. These tandem arrangements of several identical or different 3'UTR elements are referred to in this text as extended long sequence elements, which are also potential high-performance 3'UTRs.

Based on the screened 3'UTR sequence elements above or their extended long sequence elements, the present application constructs an artificial nucleic acid molecule with high RNA translation performance (including high RNA translation efficiency and stability). It also provides a vector containing said artificial nucleic acid molecule, a host cell containing said vector, a pharmaceutical composition and a kit comprising the aforementioned substances, and their use in the preparation of drugs for DNA or RNA-based therapies. Additionally, it offers the use of the above-mentioned 3'UTR sequence elements for translating the coding region of a nucleic acid molecule into a polypeptide or protein encoded by said coding region, as well as methods for obtaining RNA, polypeptides, or proteins.

Specifically, the present application provides the following technical solutions.

In a first aspect, the present application provides an artificial nucleic acid molecule comprising:
(a) a coding region of a polypeptide or protein; and
(b) at least one 3'-untranslated region (UTR) element, wherein the 3'-UTR element comprises or consists of the following nucleic acid sequences:
   an RNA sequence as shown in any one of SEQ ID Nos.1 to 23, 50 to 53, or an extended long sequence thereof, or a corresponding DNA sequence thereto.

In the above-mentioned artificial nucleic acid molecules, as preferred, the extended long sequence is an RNA sequence formed by linking multiple identical or different RNA sequences selected from those shown in SEQ ID Nos.1 to 23, 50 to 53 in tandem, either directly or via a linker, or a corresponding DNA sequence thereof.

Preferably, the term "multiple" refers to 2 to 10, more preferably 2 to 5, and even more preferably 2 to 3;
Preferably, the extended long sequence is selected from the following sequences:
an RNA sequence as shown in any one of SEQ ID NOs: 24 to 44, 49, or an extended long sequence thereof, or a corresponding DNA sequence thereto.

As preferred, the 3'-untranslated region (UTR) element comprises or consists of the following nucleic acid sequences:
an RNA sequence as shown in any one of SEQ ID NOs: 3, 8, 10, 13, 14, 24, 26, 27, 29, 33, 36, 38, 40, 44, 49 to 53, or an extended long sequence thereof, or a corresponding DNA sequence thereto.

Preferably, said artificial nucleic acid molecule further comprises:
(c) a promoter; and/or,
(d) at least one 5'-untranslated region (5'-UTR) element.

Preferably, under the control of (c) promoter, (a) coding region and (b) 3'-untranslated region element, and optionally (d) 5'-untranslated region element, can be transcribed to produce one transcript, wherein the nucleic acid sequence transcribed from (b) 3'-untranslated region element has the activity to enhance the translation efficiency and/or stability of the nucleic acid sequence transcribed from (a) coding region.

In the embodiments, said artificial nucleic acid molecule may further comprise one or more elements selected from the group consisting of: an origin of replication, an enhancer, a screening marker, and a restriction enzyme site.

In the embodiments, said artificial nucleic acid molecule may be a DNA molecule or an RNA molecule;
Preferably, the RNA molecule may be an mRNA, a self-replicating RNA, or a circular RNA; when said artificial nucleic acid molecule is an mRNA molecule, the mRNA molecule preferably comprises a polyadenylate tail at the 3' end; preferably, the polyadenylate tail has a length of 20 to 200 nucleotides, more preferably 60 to 150 nucleotides.

In feasible embodiments, the coding region encodes at least one polypeptide or protein; preferably, the polypeptide or protein is a therapeutic and/or prophylactic polypeptide or protein.

In a second aspect, the present application provides a vector comprising the artificial nucleic acid molecule as described above in the first aspect.

In a third aspect, the present application provides a host cell comprising the vector as described above in the second aspect.

In a fourth aspect, the present application provides a pharmaceutical composition, wherein the pharmaceutical composition comprises the artificial nucleic acid molecule as described in the first aspect or the vector as described in the second aspect and/or the host cells as described in the third aspect, and a pharmaceutically acceptable carrier.

In preferred embodiments, said pharmaceutical composition is used for DNA or RNA-based therapy.

In a fifth aspect, the present application provides a use of the artificial nucleic acid molecule as described in the first aspect, the vector as described in the second aspect, the host cell as described in the third aspect, and/or the pharmaceutical composition described in the fourth aspect, in the preparation of a DNA- or RNA-based drugs.

In a sixth aspect, the present application provides a kit comprising the artificial nucleic acid molecules as described in the first aspect, the vector as described in the second aspect, the host cells as described in the third aspect and/or the pharmaceutical composition described in the fourth aspect.

In a seventh aspect, the present application provides a use of one or more 3' non-translational region elements as defined in the first aspect above for translating the coding region of a nucleic acid molecule into a polypeptide or protein encoded by the coding region.

In an eighth aspect, the present application provides a use of one or more 3'-untranslated region elements as defined in the first aspect for improving the translational performance to translate the coding region of a nucleic acid molecule into a polypeptide or protein encoded by said coding region;

Preferably, the enhancement in translational performance refers to an increase in RNA translation efficiency and/or stability; more preferably, the enhancement in RNA translation efficiency is reflected in the increased total expression level of the polypeptide or protein. It indicates that the enhancement of RNA translation performance by the 3'-untranslated region elements is not transient but rather an effect that increases the overall protein enrichment.

In a ninth aspect, the present application provides a method for obtaining RNA, which includes:
(i) providing the artificial nucleic acid molecule as described in the first aspect, and
(ii) using the artificial nucleic acid molecule as a template for transcription or replication to obtain RNA.

In a tenth aspect, the present application provides a method for obtaining a polypeptide or protein, which includes:
(i) obtaining RNA encoding the polypeptide or protein according to the method described in the eighth aspect above, and
(ii) translating said RNA.

### BENEFICIAL EFFECTS

The artificial nucleic acid molecule of the present application, comprising one or more specific 3'UTR sequence elements, exhibits enhanced RNA translation performance (including higher RNA translation efficiency and enhanced RNA stability). Moreover, the enhancement in RNA translation performance has been validated across different coding sequences (CDS) and expression systems, demonstrating its excellent universality. Given the above, the artificial nucleic acid molecule holds significant potential for application in the development of RNA-based therapeutic drugs.

### BRIEF DESCRIPTION OF THE DRAWINGS

One or more embodiments are exemplarily illustrated by the figures in the corresponding drawings, which are not intended to limit the embodiments. The term "exemplary" is used herein to mean "serving as an example, embodiment, or illustration". Any embodiment described herein as "exemplary" should not be construed as superior or better than other embodiments.

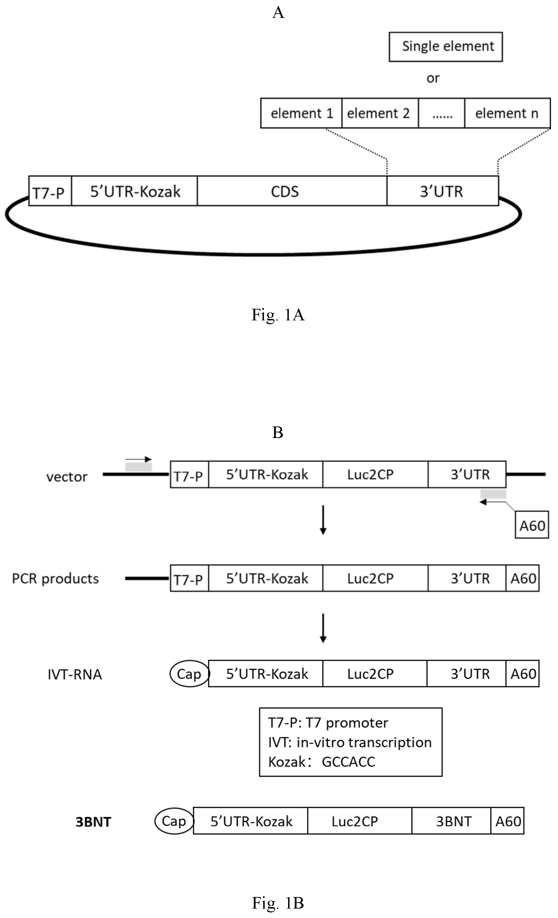

Figure 1 shows a schematic diagram of the Luc2CP vector (A) and the procedure for obtaining IVT-RNA based on this vector (B) described in Example 2. Panel A shows that one or more elements are cloned into the vector as 3 'UTR; Panel B shows PCR amplification using the vector as a template with upstream primers paired to the vector sequence upstream of T7 promoter and downstream primers containing a poly-thymidine extension paired with the downstream of 3' UTR, followed by *in vitro* transcription (IVT) of the PCR products to produce sample IVT-RNA; additionally, panel B also illustrates the schematic of the control 3BNT mRNA.

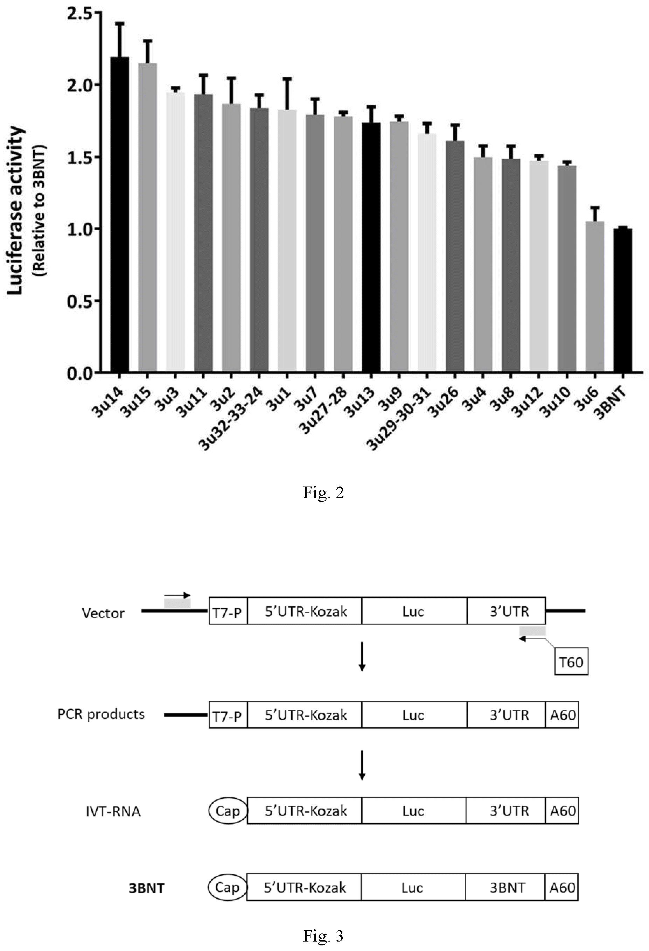

Figure 2 shows representative results of luciferase activity 24 hours post transfecting HEK293 cells with Luc2CP reporter mRNA containing different 3'UTRs, as described in Example 2. The x-axis shows the specific 3' UTR elements, and the y-axis shows luciferase activity relative to the control 3BNT. The values are presented as mean ± SEM, n=3-6.
Figure 3 shows the procedure for obtaining IVT-RNA based on Luc vector as described in Example 3. The figure illustrates that using the Luc vector as a template, PCR amplification is performed with upstream primers paired with the vector sequence upstream of the T7 promoter and downstream primers containing a poly-thymidine extension paired with the downstream of 3' UTR. The PCR products are then used as templates for IVT to generate sample IVT-RNA. Additionally, the figure also displays the schematic of the control 3BNT mRNA.

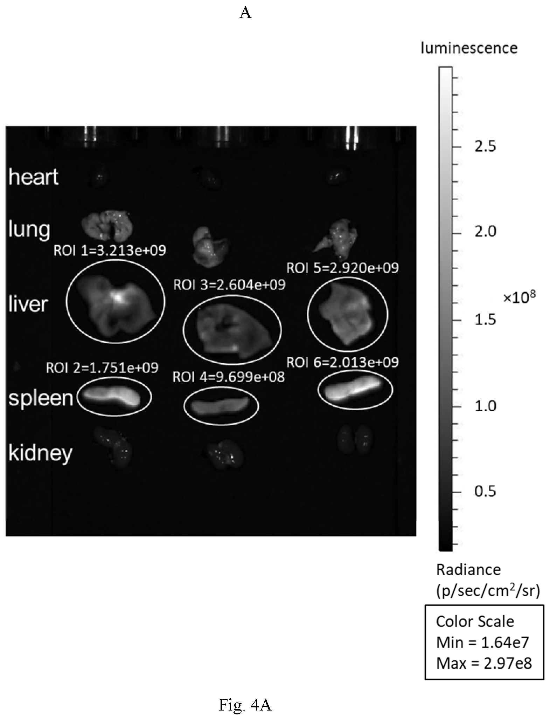

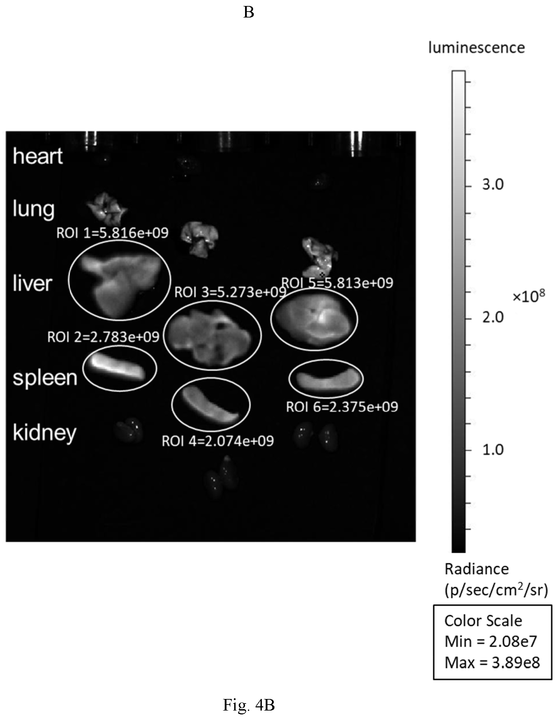

Figure 4 shows the bioluminescence imaging of *ex vivo* organs (heart, lung, liver, spleen, kidney) of mice at 4 hours post-injection with LNP-encapsulated Luc reporter mRNA containing different 3 'UTRs, as described in Example 3. Panel A shows the *ex vivo* organ imaging of Luc reporter mRNA with 3BNT as the 3' UTR. Panel B shows the *ex vivo* organ imaging of Luc reporter mRNA with 3u11 as the 3' UTR.

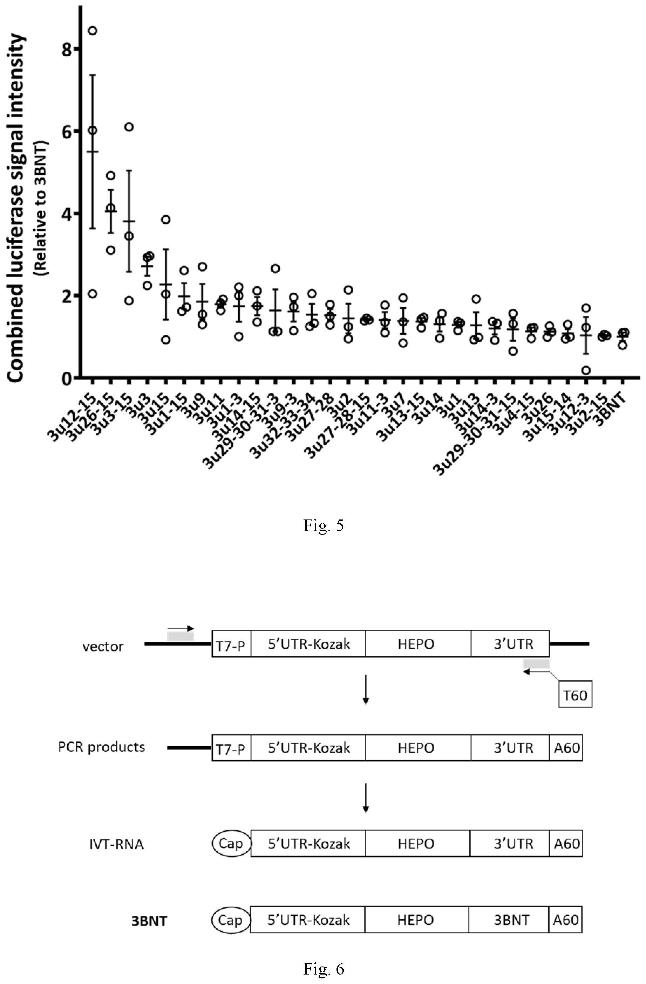

Figure 5 presents the combined luciferase signal intensity from liver and spleen in *ex vivo* organ imaging at 4 hours post-injection with LNP-encapsulated Luc reporter mRNAs containing different 3'UTRs, as described in Example 3. The x-axis indicates the specific 3'UTR elements. The y-axis shows the fold change of the combined luciferase signal intensity from liver and spleen relative to the control (3BNT). Values are presented as mean ± SEM, with n = 3 mice per group.
Figure 6 illustrates the procedure for generating IVT-RNA based on HEPO vector as described in Example 4. The figure illustrates that using HEPO vector as a template, PCR amplification was performed with upstream primers complementary to the vector sequence upstream of T7 promoter and downstream primers containing a poly-thymidine extension paired with downstream of 3' UTR. The PCR product is subsequently used as a template for IVT to produce the IVT-RNA sample. Additionally, the figure also displays the schematic of the control 3BNT mRNA.

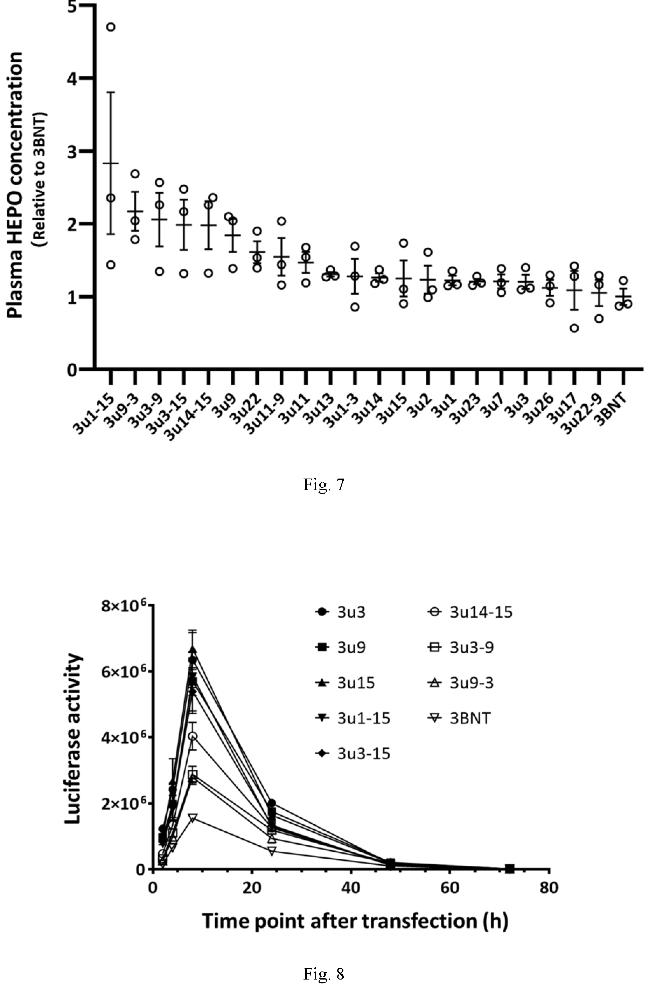

Figure 7 presents representative results of plasma HEPO levels of mice at 6 hours post-injection with LNP-encapsulated HEPO reporter mRNAs containing different 3'UTRs, as described in Example 4. The x-axis indicates the specific 3'UTR elements, and Y-axis indicates the plasma HEPO levels relative to control (3BNT). Values are presented as mean ± SEM, with n = 3 mice per group.
Figure 8 shows the representative results of luciferase activity 2, 4, 8, 24,48, and 72 h post transfecting HEK293 cells with Luc2CP reporter mRNA containing different 3' UTRs, as described in Example 5. The x-axis indicates different time points post transfection, and the y-axis shows luciferase activity. The values are presented as mean ± SEM, n=3.

### Detailed description of the invention

In order to make the purpose, technical solutions and advantages of the embodiments of the present application clearer, the technical solutions in the embodiments of the present application will be described clearly and completely below. Obviously, the described embodiments are part of the embodiments of the present application, not all the embodiments. Based on the embodiments in the present application, all other embodiments obtained by those skilled in the art without creative work are within the scope of protection of the present application.

In addition, in order to illustrate the present application better, many specific details are given in the specific implementation methods below. Those skilled in the art should understand that the present application can also be implemented without certain specific details. In some embodiments, raw materials, elements, methods, means, etc. that are familiar to those skilled in the art are not described in detail in order to highlight the main purpose of the present application.

Unless otherwise expressly stated, throughout the specification and claims, the term "including" or its variations such as "include" or "includes" will be understood to include the stated elements or components, without excluding other elements or other components.

### Example 1: Screening of universal 3'UTR sequence elements that enhance RNA translation performance

In order to obtain new 3'UTR sequence elements that stabilize mRNA, we used mouse bone marrow-derived dendritic cells (BMDCs) as the environment for screening the *in vitro* transcribed (IVT) RNAs, and the starting 3'UTR library was constructed using mRNA fragments derived from human skeletal muscle cells (HSkMC), human peripheral blood immature dendritic cells (hDC), and human embryonic kidney cells (HEK293). Before RNA extraction, the cells were grown in the presence of actinomycin D (ActD) for 3 hours to pre-select the stable RNAs. Then, the remaining mRNAs were extracted, cleaved into fragments of 50-200 nucleotides, reverse-transcribed, and cloned as 3'UTRs into a vector containing a T7 promoter (T7-P), a 5'UTR-Kozak, and a reporter gene (d2EGFP, a destabilized form of enhanced green fluorescent protein). PCR amplification is performed with upstream primers paired with the vector sequence upstream of the T7 promoter and downstream primers containing a poly-thymidine extension paired with the downstream of 3' UTR. The PCR products are then used as templates for IVT to generate mRNA with a poly-A tail (A60). Next, mRNAs were subjected to the following screening process in BMDCs: mRNAs containing the 3'UTR library were electroporated into BMDCs, and RNA was extracted at specific time points. The cDNAs were synthesized through reverse transcription, followed by PCR amplification of DNA sequences containing a T7 promoter (T7-P), a 5'UTR-Kozak, a reporter gene, the 3'UTR library, and a A60 using specific primers. The PCR-amplified products were then used as IVT templates for the next round of screening. For the cDNAs obtained in the last round of screening, the 3'UTR regions were amplified with specific primers and inserted into plasmids. Recombinant plasmids were transformed into *Escherichia coli*, and single clones were selected for sequencing and analysis of the 3'UTR regions. The obtained sequences were subjected to BLAST analysis in NCBI to identify their genomic origins. It was found that 23 core elements derived from the following genes appeared frequently after several rounds of screening (written in the format of "source gene (element code, abbreviation, reference NCBI number)", see Table 1): interferon related developmental regulator 2 (3u1, IFRD2, NM_006764.5), S100 calcium binding protein A6 (3u2, S100A6, NM_014624.4), thrombospondin 1 (3u3, THBS1, NM_003246.4), collagen type VI alpha 3 chain (3u4, COL6A3, NM_004369.4), exocyst complex component 7 (3u6, EXOC7, NM_001375975.1), lysosomal protein transmembrane 5 (3u7, LAPTM5, NM_006762.3), structural maintenance of chromosomes 1A (3u8, SMC1A, NM_006306.4), lysyl oxidase like 1 (3u9, LOXL1, NM_005576.4), phosphatidyl glycerophosphate synthase 1 (3u10, PGS1, XM_054317868.1), ribosomal protein L6 (3u11, RPL6, NM_001320137.2), ferritin heavy chain 1 (3u12, FTH1, NM_002032.3), inositol polyphosphate-5-phosphatase (3u13, OCRL, NM_001587.4), ferritin light chain (3u14, FTL, NM_000146.4), cathepsin K (3u15, CTSK, NM_000396.4), thioredoxin interacting protein (3u26, TXNIP, NM_001313972.2), SRP receptor subunit α (3u27, SRPRA, NM_003139.4), eukaryotic translation initiation factor 2 subunit β (3u28, EIF2S2, NM_001316363.2), acid phosphatase 5 (3u29, ACP5, NM_001111035.3), prosaposin (3u30, PSAP, NM_001042466.3), membrane bound transcription factor peptidase site 2 (3u31, MBTPS2, NM_015884.4), actin β (3u32, ACTB, NM_001101.5), RAF proto-oncogene, serine/threonine kinase (3u33, RAF1, NM_001354695.3), ribosomal protein S9 (3u34, RPS9, NM_001321701.2). Therefore, they are expected to serve as universal 3'UTR sequence elements which enhance RNA translation performance.

**Table 1 The screened 3'UTR elements**

| **Elem ent code** | **Source gene (homo sapiens)** | **Abbrevi ation** | **Reference NCBI number** | **Source region** |
|---|---|---|---|---|
| 3u1 | Interferon related developmental regulator 2 | IFRD2 | NM_006764 .5 | Part of CDS |
| 3u2 | S100 calcium binding protein A6 | S100A6 | NM_014624 .4 | Part of CDS |
| 3u3 | Thrombospondin 1 | THBS1 | NM_003246 .4 | Part of CDS |
| 3u4 | Collagen type VI alpha 3 chain | COL6A 3 | NM_004369 .4 | Part of CDS |
| 3u6 | Exocyst complex component 7 | EXOC7 | NM_001375 975.1 | Part of CDS + Part of 3'UTR |
| 3u7 | Lysosomal protein transmembrane 5 | LAPTM 5 | NM_006762 .3 | Part of 5'UTR + Part of CDS |
| 3u8 | Structural maintenance of chromosomes 1A | SMC1A | NM_006306 .4 | Part of 3'UTR |
| 3u9 | Lysyl oxidase like 1 | LOXL1 | NM_005576 .4 | Part of CDS |
| 3u10 | Phosphatidyl glycerophosphate synthase 1 | PGS1 | XM_054317 868.1 | Part of 3'UTR |
| 3u11 | Ribosomal protein L6 | RPL6 | NM_001320 137.2 | Part of CDS |
| 3u12 | Ferritin heavy chain 1 | FTH1 | NM_002032 .3 | Part of 3'UTR |
| 3u13 | Inositol polyphosphate-5-phosphatase | OCRL | NM_001587 .4 | Part of CDS |
| 3u14 | Ferritin light chain | FTL | NM_000146 .4 | Part of 5'UTR |
| 3u15 | Cathepsin K | CTSK | NM_000396 .4 | Part of CDS |
| 3u26 | Thioredoxin interacting protein | TXNIP | NM_001313 972.2 | Part of CDS |
| 3u27 | SRP receptor subunit α | SRPRA | NM_003139 .4 | Part of 3'UTR |
| 3u28 | Eukaryotic translation initiation factor 2 subunit β | EIF2S2 | NM_001316 363.2 | Part of 3'UTR |
| 3u29 | Acid phosphatase 5 | ACP5 | NM_001111 035.3 | Part of CDS |
| 3u30 | Prosaposin | PSAP | NM_001042 466.3 | Part of CDS |
| 3u31 | Membrane bound transcription factor peptidase site 2 | MBTPS 2 | NM_015884 .4 | Part of 3'UTR |
| 3u32 | Actin β | ACTB | NM_001101 .5 | Part of 5'UTR + Part of CDS |
| 3u33 | RAF proto-oncogene, serine/threonine kinase | RAF1 | NM_001354 695.3 | Part of 3'UTR |
| 3u34 | Ribosomal protein S9 | RPS9 | NM_001321 701.2 | Part of CDS |

For simplicity, the elements codes in Table 1 are used to refer to these 3'UTR sequence elements in the following text.

As shown in Table 1, these sequence elements are not only from the 3'UTR region of the natural genes, but also from the CDS, 5'UTR, 5'UTR+CDS or 3'UTR+CDS region of the natural genes.

In addition, the inventors also found that the truncated forms of some of the above 3'UTR elements (for example, 3u35, 3u36, 3u37, 3u38, which are the truncated versions of 3u9), serving as 3'UTR elements, can also significantly enhance the translation performance of the RNA molecules, thus they are also within the scope of protection of the present application.

In addition to using a single 3'UTR element or a truncated version thereof as the 3'UTR to enhance the translation performance of the RNA molecules, the inventors also found that the translation performance of RNA molecules can also be enhanced through using a tandem arrangement of several identical or different 3'UTR elements or truncated versions thereof. The tandem arrangement of several identical or different 3'UTR elements or truncated versions thereof are referred to herein as extended long sequence elements of the above sequence elements, which are also potential high-performance 3'UTRs. For simplicity, the tandem arrangement of elements codes is used hereinafter to refer to the extended long sequence elements. For example, 3u29-30-31 refers to the extended long sequence element based on the tandem arrangement of three elements 3u29, 3u30, and 3u31.

### Example 2: Evaluating the effect of 3'UTR sequence elements on the in vitro translation performance of mRNAs in HEK 293 cells, using a reporter CDS encoding a destabilized form of luciferase protein (Luc2CP)

In order to characterize the effect of the 3'UTR sequence elements screened in Example 1 on the *in vitro* translation performance of mRNAs, in this example, a series of 3'UTR sequence elements screened in Example 1 or extended long sequence elements thereof were cloned as 3'UTR into a vector carrying a luciferase reporter gene, and the translation performance of the reporter mRNA can be assessed following its transfection into the cells.

The reporter gene used in this experiment is a destabilized form of the luciferase (Luc2CP) gene with protein half-life of only 2 h, which facilitates the detection of mRNA stability difference through monitoring the protein expression. Meanwhile the mRNA carrying the 3'UTR element AES-mtRNR1 from BioNTech's patent CN108291230 (herein numbered 3BNT) was used as the control mRNA. The translation performance of mRNAs with different 3'UTR sequence elements was compared with that of the control mRNA with 3BNT.

Starting from a vector containing a T7 promoter (T7-P, whose corresponding mRNA sequence is shown in SEQ ID NO:54), a 5'UTR-kozak (whose corresponding mRNA sequence is shown in SEQ ID NO:55), a Luc2CP coding sequence (whose corresponding mRNA sequence is shown in SEQ ID NO:45), and a 3'UTR (whose schematic diagram is shown in Figure 1A), the transcription template DNAs were obtained through PCR amplification using a 5' primer that matched the vector sequence upstream of the T7 promotor and a 3' primer that could introduce a poly-A tail (A60) tail. The schematic diagram of the Luc2CP vector and the procedure for obtaining the IVT-RNA based on the vector is shown in Figure 1B. The PCR fragments were separated by agarose gel electrophoresis, and the target PCR fragments were recovered from the gel using the FastPure Gel DNA Extraction Mini Kit (Vazyme). Then, the nuclease that might contaminate the target PCR fragments was removed through proteinase K (Beyotime) treatment, and then the enzymatic reaction system was removed using GeneJET PCR Purification Kit (Thermo Scientific). The target PCR fragment was eluted with enzyme-free water and used as the IVT template of the corresponding RNA. The IVT reaction system (see Table 2 for details) contained the T7 RNA transcriptase mixture (purchased from Suzhou Novoprotein Scientific Inc.), the corresponding reaction buffer, 7.5 mM NTP (where the UTP used was the modified nucleotide N1-Me-pUTP), and 6 mM 3'-OMe-GAG cap. The IVT reaction was carried out at 37°C for 2 h, and then DNase I was added to remove the DNA template. The RNA was purified by NucleoSpin RNA Clean-up Kit (TaKaRa), and the concentration and purity of the RNA were analyzed by spectrophotometry (NanoDrop One, Thermo Scientific) and capillary electrophoresis (Agilent 5200). The purified RNA was transfected into HEK293 cells using Lipofectamine 3000 (Thermo Scientific). 24 h post transfection, the luciferase signal was detected using the One-Lumi^{™} II Firefly Luciferase Reporter Gene Assay Kit (Beyotime) in combination with a microplate reader (Varioskan LUX, Thermo Scientific). The representative results are shown in Figure 2 and Table 3.

**Table 2 The system for mRNA synthesis through IVT**

| **Enzyme-free water** | **Adding enzyme-free water to total volume of 20 µL** |
|---|---|
| ATP | 1.5 µL (final concentration 7.5 mM) |
| GTP | 1.5 µL (final concentration 7.5 mM) |
| CTP | 1.5 µL (final concentration 7.5 mM) |
| N1-Me-Pseudo UTP | 1.5 µL (final concentration 7.5 mM) |
| 3' OMe-GAG cap | 1.2 µL (final concentration 6 mM) |
| Transcription buffe (10×) | 2 µL |
| T7 RNA transcriptase mixture | 1 µL |
| DNA template | 0.5 µg (purified PCR product) |

**Table 3 Representative results of luciferase activity 24 h post transfection of the Luc2CP reporter mRNAs containing different 3'UTRs into HEK293 cells**

| **3'UTR** | **Luciferase activity (relative to 3BNT group)** |
|---|---|
| 3BNT | 1 |
| 3u6 | 1.05 |
| 3u10 | 1.44 |
| 3u12 | 1.47 |
| 3u8 | 1.49 |
| 3u4 | 1.5 |
| 3u26 | 1.61 |
| 3u29-30-31 | 1.66 |
| 3u9 | 1.74 |
| 3u13 | 1.74 |
| 3u27-28 | 1.78 |
| 3u7 | 1.79 |
| 3u1 | 1.82 |
| 3u32-33-34 | 1.84 |
| 3u2 | 1.87 |
| 3u11 | 1.93 |
| 3u3 | 1.95 |
| 3u15 | 2.15 |
| 3u14 | 2.19 |

As shown in Figure 2 and Table 3, 24 h post transfection of the reporter Luc2CP RNAs containing different 3'UTRs into HEK293 cells, the luciferase activity was enhanced in varying degrees compared with the control 3BNT. Among them, the elements 3u3, 3u11, 3u14, and 3u15 had the most significant effect on enhancing translation performance, reaching about 2 times that of the control 3BNT. The 3u14 especially shown the best enhancement effect, with the luciferase signal reaching up to 2.19 times that of the control 3BNT. In addition, 3u27-3u28, 3u29-3u30-3u31, and 3u32-3u33-3u34, which are tandem arrangement elements of two or three elements, also showed enhancement effect significantly better than 3BNT.

### Example 3. Evaluating the effect of 3'UTR sequence elements on the translation performance of RNA in mice, using a reporter CDS encoding luciferase with normal stability (Luc)

In order to verify whether the phenomenon in Example 2 is limited to HEK293 cells, in this example, the effect of the screened 3'UTR sequence elements on the *in vivo* translation performance of the mRNAs was tested. The reporter gene used in this experiment was a luciferase with normal stability (Luc). According to the method described in Example 2, an IVT template containing a T7 promoter (T7-P), a 5'UTR-kozak, a Luc coding sequence (whose corresponding mRNA sequence is shown in SEQ ID NO: 46), a 3'UTR, and an A60 tail was obtained by PCR amplification, and IVT was performed to obtain the RNA (the schematic diagrams of Luc vector and the procedure for obtaining IVT-RNA based on the vector are shown in Figure 3); the RNA was purified, and the purified RNA was encapsulated using LNPs prepared by ALC-0315, DSPC, cholesterol and ALC-0159. The mRNA concentration, encapsulation efficiency, mRNA integrity, particle size and Zeta potential of the LNP/mRNA samples were analyzed using Quant-iTTM RiboGreenTM RNA Assay Kit (Invitrogen) in combination with a microplate reader (Varioskan LUX, Thermo Scientific), capillary electrophoresis (Agilent 5200), and light scattering instrument (Zetasizer Ultra). The LNP/mRNA was injected into the ICR female mice via the tail vein, and luciferase substrate (D-Luciferin) was injected intraperitoneally 4 hours later for *in vivo* imaging; the mice were then sacrificed and *ex vivo* organ (heart, liver, spleen, lung, kidney) imaging was performed.

For the study of the effects of various 3'UTR sequence elements on the *in vivo* mRNA translation performance, the results of *in vivo* imaging and *ex vivo* organ imaging showed that the luciferase positive signal mainly presented in the liver and spleen regions, and the *ex vivo* organ imaging was less affected by the body position of the mouse. Therefore, the combination of the luciferase signals of liver and spleen in *ex vivo* organ imaging was used as the basis for evaluating the translation performance of mRNA.

As representative results of *ex vivo* organ imaging, this example only presents the *ex vivo* organ (heart, lung, liver, spleen, kidney) bioluminescent images of the control 3'UTR (i.e., 3BNT) and the representative 3'UTR of the present application (i.e., 3u11), which are shown in Figures 4A and 4B respectively. As can be seen from Figures 4A and 4B, the 3'UTR of the present application (i.e., 3u11) shown luciferase signal in each *ex vivo* organ with intensity significantly higher than that of the control 3'UTR (i.e., 3BNT).

Figure 5 and Table 4 present the representative results of the combined luciferase signals from the liver and spleen, measured 4 hours after injecting mice with Luc reporter mRNAs containing various 3'UTR sequence elements.

**Table 4. Representative results of the combined luciferase signals from liver and spleen, measured 4 h after injecting mice with Luc reporter mRNAs containing various 3'UTR sequence elements**

| **3'UTR** | **Luciferase activity (relative to 3BNT group)** |
|---|---|
| 3BNT | 1 |
| 3u2-15 | 1.03 |
| 3u12-3 | 1.04 |
| 3u15-14 | 1.08 |
| 3u26 | 1.12 |
| 3u4-15 | 1.13 |
| 3u29-30-31-15 | 1.18 |
| 3u14-3 | 1.21 |
| 3u13 | 1.28 |
| 3u1 | 1.29 |
| 3u14 | 1.31 |
| 3u13-15 | 1.38 |
| 3u7 | 1.39 |
| 3u11-3 | 1.41 |
| 3u27-28-15 | 1.42 |
| 3u2 | 1.45 |
| 3u27-28 | 1.53 |
| 3u32-33-34 | 1.54 |
| 3u9-3 | 1.61 |
| 3u29-30-31-3 | 1.64 |
| 3u14-15 | 1.74 |
| 3u1-3 | 1.74 |
| 3u11 | 1.79 |
| 3u36 | 1.84 |
| 3u9 | 1.85 |
| 3u37 | 1.91 |
| 3u38 | 1.91 |
| 3u1-15 | 1.99 |
| 3u35 | 2.03 |
| 3u15 | 2.27 |
| 3u3 | 2.71 |
| 3u3-15 | 3.81 |
| 3u26-15 | 4.05 |
| 3u12-15 | 5.5 |

Table 4 and Figure 5 demonstrate that all the tested 3'UTR sequence elements significantly outperformed the control 3BNT in enhancing RNA translation performance. More precisely, Table 4 shows that the luciferase activity of the reporter Luc RNA carrying different 3'UTR elements was enhanced to varying degrees compared to the control 3BNT. Among them, the extended long sequence elements 3u3-15, 3u26-15, and 3u12-15 exhibited the most significant effects on improving translation performance, reaching 3.8 to 5.5 times that of the control 3BNT. Among the short sequence elements, 3u3, 3u9, 3u11, 3u15, 3u35, 3u36, 3u37, and 3u38 showed the most significant effects on enhancing RNA translation performance, with a large overlap with the best-performing short sequence elements (3u3, 3u11, 3u14, and 3u15) in the Luc2CP reporter RNA.

These results suggest that the enhancement of mRNA translation performance by these 3'UTR sequence elements compared to 3BNT is not limited to specific expression systems. Their advantages are still evident in the complex multicellular in vivo environment, thus demonstrating good universality.

### Example 4. Evaluating the effect of 3'UTR sequence elements on the translation performance of RNAs in mice, using a reporter CDS encoding human erythropoietin (HEPO)

In order to verify whether the phenomenon in Example 2 has CDS sequence specificity, in this example, the effect of the screened 3'UTR sequence elements on the *in vivo* translational performance of mRNAs carrying other CDS sequences was examined. The reporter gene used in this experiment was the human erythropoietin (HEPO) gene. Based on the method described in Example 2, the IVT template containing a T7 promoter (T7-P), a 5'UTR-Kozak, a HEPO coding sequence (whose corresponding mRNA sequence is shown in SEQ ID NO:47), a 3'UTR, and an A60 tail was obtained through PCR amplification, while IVT was performed to obtain the corresponding RNA (the schematic diagram of HEPO vector and the procedure for obtaining IVT-RNA based on the vector are shown in Fig. 6); the obtained RNA was purified and then encapsulated using LNP formulated with ALC-0315, DSPC, cholesterol, and ALC-0159. The LNP/mRNA was injected into the ICR female mice via the tail vein, and 6 h later, the eyeball was removed and the blood was collected and placed in the anticoagulation tubes, gently inverted and mixed, and then centrifuged at 4°C for 10 min at 2000g. The supernatant, which was the plasma, was collected. Subsequently, the HEPO content in the plasma was detected using a HEPO ELISA kit (Elabscience).

The results are shown in Figure 7 and Table 5.

**Table 5. Representative results of plasma HEPO levels in mice 6 hours after injection of HEPO reporter mRNA containing different 3'UTRs.**

| **3'UTR** | **HEPO concentration (relative to 3BNT group)** |
|---|---|
| 3BNT | 1 |
| 3u29-30-31-9 | 1.05 |
| 3u27-28 | 1.09 |
| 3u26 | 1.12 |
| 3u3 | 1.2 |
| 3u7 | 1.21 |
| 3u32-33-34 | 1.21 |
| 3u1 | 1.22 |
| 3u2 | 1.23 |
| 3u15 | 1.25 |
| 3u14 | 1.26 |
| 3u1-3 | 1.28 |
| 3u13 | 1.31 |
| 3u11 | 1.47 |
| 3u11-9 | 1.54 |
| 3u29-30-31 | 1.61 |
| 3u9 | 1.84 |
| 3u14-15 | 1.98 |
| 3u3-15 | 1.99 |
| 3u3-9 | 2.06 |
| 3u9-3 | 2.17 |
| 3u1-15 | 2.83 |

The results in Figure 7 and Table 5 show that the HEPO protein expression of the reporter HEPO RNAs carrying different 3'UTR elements are enhanced in varying degrees, compared with the control 3BNT. Among them, 3u9, 3u14-15, 3u3-15, 3u3-9, 3u9-3, and 3u1-15 have the most significant effect on enhancing RNA translation performance, reaching 1.8-2.8 times that of 3BNT. These results indicate that in terms of enhancing mRNA translation performance, these 3'UTR sequence elements have good compatibility with different CDSs, among which 3u9 and 3u11 rank top on three CDSs (Luc2CP, Luc, HEPO) and different expression environments (HEK293 cells, BALB/c mice), indicating that 3u9 and 3u11 have favorable universality. However, the ranking of the translation-enhancing effects of these 3'UTR sequence elements varies in different CDS and expression environments, indicating that these sequence elements possess both universality and selectivity. In specific applications, it is necessary to select different sequence elements for different CDSs and target organs to achieve the best effect.

### Example 5. Evaluating the effect of 3'UTR sequence elements on RNA translation efficiency, functional stability, and total protein expression in cells, using a reporter CDS encoding a destabilized form of luciferase protein (Luc2CP)

In order to verify whether the phenomenon in Example 2 is limited to a certain time point after transfecting the cells, in this example, the effects of the 3'UTR sequence elements on the translation efficiency, functional stability, and total protein expression of the mRNAs in cells were detected.

The reporter gene used in this experiment was a destabilized form of luciferase (Luc2CP). According to the method described in Example 2, a IVT template containing a T7 promoter (T7-P), a 5'UTR-kozak, a Luc2CP coding sequence (whose corresponding mRNA sequence is shown in SEQ ID NO:45), a 3'UTR, and an A60 tail was obtained by PCR amplification, and IVT was performed to obtain RNA (the schematic diagrams of the Luc2CP vector and the procedure for obtaining IVT-RNA based on the vector are shown in Figure 1B). The RNA was purified by NucleoSpin RNA Clean-up Kit (TaKaRa), and the concentration and purity of RNA were analyzed by spectrophotometry (NanoDrop One, Thermo Scientific) and capillary electrophoresis (Agilent 5200). The purified RNA was transfected into HEK293 cells using Lipofectamine 3000 (Thermo Scientific). 2 h after transfection of the RNA into HEK293 cells, the culture supernatant was sucked and discarded, and the fresh culture medium (DMEM + 1% two antibiotics + 10% FBS) was added. The cell samples were collected 2, 4, 8, 24, 48, and 72 h after transfection of the RNA into HEK293 cells, and the luciferase signal was detected using the One-Lumi^{™} II Firefly Luciferase Reporter Gene Assay Kit (Beyotime) in combination with microplate reader (Varioskan LUX, Thermo Scientific). The results are shown in Figure 8.

The temporal protein expression data illustrated in Figure 8 were fitted using piecewise polynomial interpolation, implemented in the R programming language. And the parameters such as RNA translation efficiency (represented by the maximum slope of the rising stage of protein expression), functional stability (represented by the time that the protein expression reached the peak) and total protein expression (represented by the area under the curve) were derived. For details on the method, please refer to Kuhn, A. N., et al. Gene therapy 17.8 (2010): 961-971.

Table 6 shows the translation efficiency of the reporter Luc2CP RNAs carrying different 3'UTR elements. It can be seen from Table 6 that the screened 3'UTR elements increase the RNA translation efficiency by 1.87-4.38 times, compared with the control 3BNT. Among which 3u9, 3u3, 3u15, and 3u1-15 have the most significant ability in enhancing the RNA translation efficiency. This result indicates that the 3'UTR element of the present invention has a significantly higher ability in enhancing RNA translation efficiency.

Table 7 shows the functional stability of the reporter Luc2CP RNAs carrying different 3'UTR elements. As shown in Table 7, the peak protein expression time of RNAs carrying the screened 3'UTR elements is slightly extended compared to the control 3BNT. This suggests that the 3'UTR elements of this invention have the effect of stabilizing RNA.

Table 8 shows the total protein expression of the reporter Luc2CP RNAs carrying different 3'UTR elements. As shown in Table 8, the total protein expression of RNA carrying the screened 3'UTR elements is increased by 1.79 to 3.78 times compared to the control 3BNT. Among them, the elements 3u1-15, 3u9, 3u15, and 3u3 show the most significant ability to enhance the total protein expression of RNA. This indicates that the 3'UTR elements of this invention can significantly improve the translation performance of RNA.

The above results demonstrate that the 3'UTR sequence elements of the present invention do not merely enhance mRNA translation performance at a single time point. Instead, their impact is more comprehensively manifested through a sustained elevation in overall protein enrichment levels.

**Table 6. Representative results of translation efficiency of Luc2CP reporter mRNAs containing different 3'UTRs in HEK293 cells**

| **3'UTR** | **RNA translation efficiency (relative to 3BNT group)** |
|---|---|
| 3BNT | 1 |
| 3u3-9 | 1.87 |
| 3u9-3 | 1.91 |
| 3u14-15 | 2.88 |
| 3u3-15 | 3.77 |
| 3u9 | 4.03 |
| 3u3 | 4.23 |
| 3u15 | 4.26 |
| 3u1-15 | 4.38 |

**Table 7. Representative results of functional stability of Luc2CP reporter mRNAs containing different 3'UTRs in HEK293 cells**

| **3'UTR** | **RNA functional stability (relative to 3BNT group)** |
|---|---|
| 3u15 | 11.8 |
| 3BNT | 11.9 |
| 3u3-15 | 12.1 |
| 3u1-15 | 12.1 |
| 3u9-3 | 12.2 |
| 3u14-15 | 12.2 |
| 3u9 | 12.2 |
| 3u3 | 12.2 |
| 3u3-9 | 12.3 |

**Table 8. Representative results of total protein translation of Luc2CP reporter mRNAs containing different 3'UTRs after transfecting HEK293 cells**

| **3'UTR** | **Total protein expression of RNA (relative to 3BNT group)** |
|---|---|
| 3BNT | 1 |
| 3u9-3 | 1.79 |
| 3u3-9 | 1.96 |
| 3u14-15 | 2.41 |
| 3u3-15 | 2.96 |
| 3u1-15 | 3.11 |
| 3u9 | 3.42 |
| 3u15 | 3.67 |
| 3u3 | 3.78 |

Finally, it should be noted that the above embodiments are only used to illustrate the technical solutions of the present application, rather than to limit them; although the present application is described in detail with reference to the above embodiments, those skilled in the art should understand that they can still modify the technical solutions recorded in the above embodiments, or replace some of the technical features therein with equivalents; and these modifications or replacements do not make the essence of the corresponding technical solutions deviate from the spirit and scope of the technical solutions of the present application.

### Industrial Applicability

The 3'UTR sequence elements for mRNA provided in the present application can significantly enhance the translation performance of the mRNA molecules, including enhancing RNA translation efficiency and RNA stability. Moreover, this improvement demonstrates excellent universality, being applicable across different CDSs and expression systems. These advancements substantially improve the druggability of mRNA therapeutics and hold significant importance for the development and application of nucleic acid-based prophylactics and therapeutics.

Sequences in present application:

| SEQ ID NO. | Description | Sequence |
|---|---|---|
| 1 | 3u1 | GCCUGCUCUGCGUGCAGCUGGGCCCUGGACCUAAGGGUG AGGAGCUGUUCACAGCCUGCAGCCUCUGCUGGUCUCUG UGCUCAGUGACAGCACAGCUAGCCCUGCUGCCCGGCUCCA CUGUGCUUCUGC |
| 2 | 3u2 | CCGGUCCAAGUCUUCCAUCAGCCUUGCAAUUUCAGCAUCC UGCAGCUUCGAGCCAAUGGUGAGCUCCUUCUGGAUCAGC UCCUUCAGCUCCUUCUUGCUCAGGGUGUGCUUGUCACAC U |
| 3 | 3u3 | GGGUCAUGCCACAGGGUGCGCACCUGGCCAGGGGUGUUU CCUGUGUGCCACAGGGCGUUCCGCAGGUGCUCGCCAGGCC CUGUGGUGGAGUUUACAACUUUCAC |
| 4 | 3u4 | CGUUUGUGGCCAGGAACACAUUUAAGCGUGUGAGGAACG GAUUCCUAAUGAGGAAAGUGGCUGUUUCUUCAGCAACA CACCCACAAGAGCAUCCCCACA |
| 5 | 3u6 | GAAGUACAUCAAGUACGGGGUGGAGCAGGUGGGCGACAU GAUCGAUCGCCUUUUCGACACCUCUGCCUGAGCCUGCUGC UAGCCCUGCCUGGUUCCACC |
| 6 | 3u7 | GGCGGUGGUUGCGAUGCGGACAUUGAAGCAGCAGCAGGU CUGGCGGACAGUGGACAAGCGGGGUCCAUGGUGCUGCC GUCCCCUCCUCUGAGACACU |
| 7 | 3u8 | CCCGGCUUUGCUGCCUCUGCAUGAUCCUUAUAGCUAAUG AGGUUAGUUCCUAAAAUGAACCUCACAGGUGUUAGGCUG GGAUGUGGCCCAGGAUAC |
| 8 | 3u9 | GGCUGAACUCGUCCAUGCUGUGGUAAUGCUGGUGGCAGC UGUGCCACUCCCAGGUGUGCCGUGGCCGGUUGGGGAGGA AGUCUGCUGUGCCCUGG |
| 9 | 3ul0 | GCCAGGCACUAAGGGAGCCCACGCUGCACACAUGAGAUG CCGGCACUAGCGCAUGUGCCGGCCACAUGGGUCCUGGCUC AUCUUUGACUCCUCAC |
| 10 | 3u11 | UGUGCCUCGAAAGCUGUUGAGCCACGGCAAAAAAACCCU UCAGUCAGCACGUGAGAAAACUGCGAGCCAGCAUUACCC CCGGGACCAUUCUGAU |
| 11 | 3u12 | AAUUUCCCCAUAGCCGUGGGGUGACUUCCCUGGUCACCA AGGCAGUGCAUGCAUGUUGGGGUUCCUUUACCUUUUCU AUAAGUUGUAC |
| 12 | 3u13 | CUUAUCUUCUCCCGAGUUCAGGAUGGUUACAGAGUCUUU GCUGACAUACACAUCAAGAGAAAUGUCCACUGUCUCAUU UGGCUCCAAG |
| 13 | 3u14 | GCGGAGAGGAAAUCGGAGGGCGGUCGGAGGCUGGAAGAG AGUCCCCGGAUCUGUUCCGUCCAAACACUGUUGAAGCAA GAGACAGACC |
| 14 | 3u15 | UGAGAAAGCCCUGAAGAGGGCAGUGGCCCGAGUGGACC UGUCUCUGUGGCCAUUGAUGCAAGCCUGACCUCCUUCCA GUUUUACA |
| 15 | 3u26 | AUCCAUUUUCUCAGAUCUCCUUGAAAUUGAGGCCUUUU CGAUAGUUUCGGGUCAGGUAAAAAUGGCCUCCUGGCGUA AGCUUUUCAAGGUUUUUUGGAGGCUUUUUGUAAAUUGUG AUAGGAACUUUGGACCUUGAACUUACGUAUCAUGUGGAG AAGAGCCAAUUUAACAAACUAGGAA |
| 16 | 3u27 | GAAGUAAGCUCUGCAUGUUCCCCUUCCUGGGAGGAAACC AAUUGUCAGA |
| 17 | 3u28 | GGCUGGACAGGUUUGCCAUCAGAGUGGAUAUACCG |
| 18 | 3u29 | GUGACAGUCAUCUCUUUGGAGCUGAUCUCCACAUAGGCA AAGCCACCCAGUGAGACU |
| 19 | 3u30 | GGAGAAAUGAGCCGUCCUGGGGAGGUGUGCUCUGCUCUC AACCUCUGCGAGUC |
| 20 | 3u31 | CCGGUUUUAUACAAAUGGUACUGAUCUGCUCUUUCCUAG UAAAGUGGUU |
| 21 | 3u32 | GAGCCGUUGUCGACGACGAGCGCGGCGAUAUCAUCAUCC AUGGUGAGCU |
| 22 | 3u33 | AGAAGGAAGCCAUUUGCAGUGCUGGUGUCCUGCUCCC UCCCCACA |
| 23 | 3u34 | AUUUACCCUGGCCAAGAUCCGCAAGGCCGCCCGGGAACU GCUGACGC |
| 24 | 3u1-15 | GCCUGCUCUGCGUGCAGCUGGGCCCUGGACCUAAGGGUG AGGAGCUGUUUCACAGCCUGCAGCCUCUGCUGGUCUCUG UGCUCAGUGACAGCACAGCUAGCCCUGCUGCCCGGCUCCA CUGUGCUUCUGCUGAGAAAGCCCUGAAGAGGGCAGUGGC CCGAGUGGGACCUGUCUCUGUGGCCAUUGAUGCAAGCCU GACCUCCUUCCAGUUUACA |
| 25 | 3u2-15 | CCGGUCCAAGUCUUCCAUCAGCCUUGCAAUUUCAGCAUCC UGCAGCUUCGAGCCAAUGGUGAGCUCCUUCUGGAUCAGC UCCUUCAGCUCCUUCUUGCUCAGGGUGUGCUUGUCACAC UUGAGAAAGCCCUGAAGAGGGCAGUGGCCCGAGUGGGAC CUGUCUCUGUGGCCAUUGAUGCAAGCCUGACCUCCUUCC AGUUUACA |
| 26 | 3u3-9 | GGGUCAUGCCACAGGGUGCGCACCUGGCCAGGGGUGUUU CCUGUGUGCCACAGGGCGUUCCGCAGGUGCUCGCCAGGCC CUGUGGUGGAGUUUACAACUUUCACGGCUGAACUCGUCC AUGCUGUGGUAAUGCUGGUGGCAGCUGUGCCACUCCCAG GUGUGCCGUGGCCGGUUGGGGAGGAAGUCUGCUGUGCCC UGG |
| 27 | 3u3-15 | GGGUCAUGCCACAGGGUGCGCACCUGGCCAGGGGUGUUU CCUGUGUGCCACAGGGCGUUCCGCAGGUGCUCGCCAGGCC CUGUGGUGGAGUUUACAACUUUCACUGAGAAAGCCCUGA AGAGGGCAGUGGCCCGAGUGGGACCUGUCUCUGUGGCCA UUGAUGCAAGCCUGACCUCCUUCCAGUUUACA |
| 28 | 3u4-15 | CGUUUGUGGCCAGGAACACAUUUAAGCGUGUGAGGAACG GAUUCCUAAUGAGGAAAGUGGCUGUUUCUUCAGCAACA CACCCACAAGAGCAUCCCCACAUGAGAAAGCCCUGAAGA GGGCAGUGGCCCGAGUGGGACCUGUCUCUGUGGCCAUUG AUGCAAGCCUGACCUCCUUCCAGUUUACA |
| 29 | 3u9-3 | GGCUGAACUCGUCCAUGCUGUGGUAAUGCUGGUGGCAGC UGUGCCACUCCCAGGUGUGCCGUGGCCGGUUGGGGAGGA AGUCUGCUGUGCCCUGGGGGUCAUGCCACAGGGUGCGCA CCUGGCCAGGGGUGUUCCUGUGUGCCACAGGGCGUUCC GCAGGUGCUCGCCAGGCCCUGUGGUGGAGUUUACAACUU UCAC |
| 30 | 3u11-3 | UGUGCCUCGAAAGCUGUUGAGCCACGGCAAAAAAACCCU UCAGUCAGCACGUGAGAAAACUGCGAGCCAGCAUUACCC CCGGGACCAUUCUGAUGGGUCAUGCCACAGGGUGCGCAC CUGGCCAGGGGUGUUCCUGUGUGCCACAGGGCGUUCCG CAGGUGCUCGCCAGGCCCUGUGGUGGAGUUUACAACUUU CAC |
| 31 | 3u11-9 | UGUGCCUCGAAAGCUGUUGAGCCACGGCAAAAAAACCCU UCAGUCAGCACGUGAGAAAACUGCGAGCCAGCAUUACCC CCGGGACCAUUCUGAUGGCUGAACUCGUCCAUGCUGUGG UAAUGCUGGUGGCAGCUGUGCCACUCCCAGGUGUGCCGU GGCCGGUUGGGGAGGAAGUCUGCUGUGCCCUGG |
| 32 | 3u12-3 | AAUUUCCCCAUAGCCGUGGGUGACUUCCCUGGUCACCA AGGCAGUGCAUGCAUGUUGGGUUUCCUUUACCUUUUCU AUAAGUUGUACGGGUCAUGCCACAGGGUGCGCACCUGGC CAGGGGUGUUCCUGUGUGCCACAGGGCGUUCCGCAGGU GCUCGCCAGGCCCUGUGGUGGAGUUUACAACUUUCAC |
| 33 | 3u12-15 | AAUUUCCCCAUAGCCGUGGGGUGACUUCCCUGGUCACCA AGGCAGUGCAUGCAUGUUGGGGUUUCCUUUACCUUUUCU AUAAGUUGUACUGAGAAAGCCCUGAAGAGGGCAGUGGCC CGAGUGGGACCUGUCUCUGUGGCCAUUGAUGCAAGCCUG ACCUCCUUCCAGUUUUACA |
| 34 | 3u13-15 | CUUAUCUUCUCCCGAGUUCAGGAUGGUUACAGAGUCUUU GCUGACAUACACAUCAAGAGAAAUGUCCACUGUCUCAUU UGGCUCCAAGUGAGAAAGCCCUGAAGAGGGCAGUGGCCC GAGUGGGACCUGUCUCUGUGGCCAUUGAUGCAAGCCUGA CCUCCUUCCAGUUUUACA |
| 35 | 3u14-3 | GCGGAGAGGAAAUCGGAGGGCGGUCGGAGGCUGGAAGAG AGUCCCCGGAUCUGUUCCGUCCAAACACUGUUGAAGCAA GAGACAGACCGGGUCAUGCCACAGGGUGCGCACCUGGCC AGGGGUGUUUCCUGUGUGCCACAGGGCGUUCCGCAGGUG CUCGCCAGGCCCUGUGGUGGAGUUUACAACUUUCAC |
| 36 | 3u14-15 | GCGGAGAGGAAAUCGGAGGGCGGUCGGAGGCUGGAAGAG AGUCCCCGGAUCUGUUCCGUCCAAACACUGUUGAAGCAA GAGACAGACCUGAGAAAGCCCUGAAGAGGGCAGUGGCCC GAGUGGGACCUGUCUCUGUGGCCAUUGAUGCAAGCCUGA CCUCCUUCCAGUUUUACA |
| 37 | 3u15-14 | UGAGAAAGCCCUGAAGAGGGCAGUGGCCCGAGUGGACC UGUCUCUGUGGCCAUUGAUGCAAGCCUGACCUCCUUCCA GUUUUACAGCGGAGAGGAAAUCGGAGGGCGGUCGGAGGC UGGAAGAGAGUCCCCGGAUCUGUUCCGUCCAAACACUGU UGAAGCAAGAGACAGACC |
| 38 | 3u26-15 | AUCCAUUUUCUCAGAUCUCCUUGAAAUUGAGGCCUUUU CGAUAGUUUCGGGUCAGGUAAAAUGGCCUCCUGGCGUA AGCUUUUCAAGGUUUUUGGAGGCUUUUGUAAAUUGUG AUAGGAACUUUGGACCUUGAACUUACGUAUCAUGUGGAG AAGAGCCAAUUUAACAAACUAGGAAUGAGAAAGCCCUGA AGAGGGCAGUGGCCCGAGUGGGACCUGUCUCUGUGGCCA UUGAUGCAAGCCUGACCUCCUUCCAGUUUACA |
| 39 | 3u27-28-15 | GAAGUAAGCUCUGCAUGUUCCCCUUCCUGGGAGGAAACC AAUUGUCAGAGGCUGGACAGGUUUGCCAUCAGAGUGGAU AUACCGUGAGAAAGCCCUGAAGAGGGCAGUGGCCCGAGU GGGACCUGUCUCUGUGGCCAUUGAUGCAAGCCUGACCUC CUUCCAGUUUUACA |
| 40 | 3u29-30-31 | GUGACAGUCAUCUCUUUGGAGCUGAUCUCCACAUAGGCA AAGCCACCCAGUGAGACUGGAGAAAUGAGCCGUCCUGGG GAGGUGUGCUCUGCUCUCAACCUCUGCGAGUCCCGGUUU UAUACAAAUGGUACUGAUCUGCUCUUUCCUAGUAAAGUG GUU |
| 41 | 3u29-30-31-3 | GUGACAGUCAUCUCUUUGGAGCUGAUCUCCACAUAGGCA AAGCCACCCAGUGAGACUGGAGAAAUGAGCCGUCCUGGG GAGGUGUGCUCUGCUCUCAACCUCUGCGAGUCCCGGUUU UAUACAAAUGGUACUGAUCUGCUCUUUCCUAGUAAAGUG GUUGGGUCAUGCCACAGGGUGCGCACCUGGCCAGGGGUG UUUCCUGUGUGCCACAGGGCGUUCCGCAGGUGCUCGCCA GGCCCUGUGGUGGAGUUACAACUUUCAC |
| 42 | 3u29-30-31-9 | GUGACAGUCAUCUCUUUGGAGCUGAUCUCCACAUAGGCA AAGCCACCCAGUGAGACUGGAGAAUGAGCCGUCCUGGG GAGGUGUGCUCUGCUCUCAACCUCUGCGAGUCCCGGUUU UAUACAAAUGGUACUGAUCUGCUCUUUCCUAGUAAAGUG GUUGGCUGAACUCGUCCAUGCUGUGGUAAUGCUGGUGGC AGCUGUGCCACUCCCAGGUGUGCCGUGGCCGGUUGGGGA GGAAGUCUGCUGUGCCCUGG |
| 43 | 3u29-30-31-15 | GUGACAGUCAUCUCUUUGGAGCUGAUCUCCACAUAGGCA AAGCCACCCAGUGAGACUGGAGAAAUGAGCCGUCCUGGG |
| | | GAGGUGUGCUCUGCUCUCAACCUCUGCGAGUCCCGGUUU UAUACAAAUGGUACUGAUCUGCUCUUUCCUAGUAAAGUG GUUUGAGAAAGCCCUGAAGAGGGCAGUGGCCCGAGUGGG ACCUGUCUCUGUGGCCAUUGAUGCAAGCCUGACCUCCUU CCAGUUUACA |
| 44 | 3u32-33-34 | GAGCCGUUGUCGACGACGAGCGCGGCGAUAUCAUCAUCC AUGGUGAGCUAGAAGGAAGCCAUUUGCAGUGCUGGUGUG UCCUGCUCCCUCCCCACAAUUUACCCUGGCCAAGAUCCGC AAGGCCGCCCGGGAACUGCUGACGC |
| 45 | Luc2CP_C DS | AUGGAAGAUGCCAAAAACAUUAAGAAGGGCCCAGCGCCA UUCUACCCACUCGAAGACGGGACCGCCGGCGAGCAGCUGC ACAAAGCCAUGAAGCGCUACGCCCUGGUGCCCGGCACCAU CGCCUUUACCGACGCACAUAUCGAGGUGGACAUUACCUA CGCCGAGUACUUCGAGAUGAGCGUUCGGCUGGCAGAAGC UAUGAAGCGCUAUGGGCUGAAUACAAACCAUCGGAUCGU GGUGUGCAGCGAGAAUAGCUUGCAGUUCUUCAUGCCCGU GUUGGGUGCCCUGUUCAUCGGUGUGGCUGUGGCCCCAGC UAACGACAUCUACAACGAGCGCGAGCUGCUGAACAGCAU GGGCAUCAGCCAGCCCACCGUCGUAUUCGUGAGCAAGAA AGGGCUGCAAAAGAUCCUCAACGUGCAAAAGAAGCUACC GAUCAUACAAAAGAUCAUCAUCAUGGAUAGCAAGACCGA CUACCAGGGCUUCCAAAGCAUGUACACCUUCGUGACUUC CCAUUUGCCACCCGGCUUCAACGAGUACGACUUCGUGCCC GAGAGCUUCGACCGGGACAAAACCAUCGCCCUGAUCAUG AACAGUAGUGGCAGUACCGGAUUGCCCAAGGGCGUAGCC CUACCGCACCGCACCGCUUGUGUCCGAUUCAGUCAUGCCC GCGACCCCAUCUUCGGCAACCAGAUCAUCCCCGACACCGC UAUCCUCAGCGUGGUGCCAUUUCACCACGGCUUCGGCAU GUUCACCACGCUGGGCUACUUGAUCUGCGGCUUUCGGGU CGUGCUCAUGUACCGCUUCGAGGAGGAGCUAUUCUUGCG CAGCUUGCAAGACUAUAAGAUUCAAUCUGCCCUGCUGGU GCCCACACUAUUUAGCUUCUUCGCUAAGAGCACUCUCAU CGACAAGUACGACCUAAGCAACUUGCACGAGAUCGCCAG CGGCGGGGCGCCGCUCAGCAAGGAGGUAGGUGAGGCCGU GGCCAAACGCUUCCACCUACCAGGCAUCCGCCAGGGCUAC GGCCUGACAGAAACAACCAGCGCCAUUCUGAUCACCCCCG AAGGGGACGACAAGCCUGGCGCAGUAGGCAAGGUGGUGC CCUUCUUCGAGGCUAAGGUGGUGGACUUGGACACCGGUA AGACACUGGGUGUGAACCAGCGCGGCGAGCUGUGCGUCC GUGGCCCCAUGAUCAUGAGCGGCUACGUUAACAACCCCG AGGCUACAAACGCUCUCAUCGACAAGGACGGCUGGCUGC ACAGCGGCGACAUCGCCUACUGGGACGAGGACGAGCACU UCUUCAUCGUGGACCGGCUGAAGAGCCUGAUCAAAUACA AGGGCUACCAGGUAGCCCCAGCCGAACUGGAGAGCAUCC UGCUGCAACACCCCAACAUCUUCGACGCCGGGGUCGCCGG CCUGCCCGACGACGAUGCCGGCGAGCUGCCCGCCGCAGUC |
| | | GUCGUGCUGGAACACGGUAAAACCAUGACCGAGAAGGAG AUCGUGGACUAUGUGGCCAGCCAGGUUACAACCGCCAAG ΛΛGCUGCGGUGGUGUUGUGUUCGUGGΛCGΛGGUGCCU AAAGGACUGACCGGCAAGUUGGACGCCCGCAAGAUCCGC GAGAUUCUCAUUAAGGCCAAGAAGGGCGGCAAGAUCGCC GUGAAUUCUGCUUGCAAGAACUGGUUCAGUAGCUUAAGC CACUUUGUGAUCCACCUUAACAGCCACGGCUUCCCUCCCG AGGUGGAGGAGCAGGCCGCCGGCACCCUGCCCAUGAGCU GCGCCCAGGAGAGCGGCAUGGAUAGACACCCUGCUGCUU GCGCCAGCGCCAGGAUCAACGUCUAAA |
| 46 | Luc_CDS | AUGGAAGAUGCCAAAAACAUCAAGAAGGGCCCGCACCC UUCUAUCCCUUGGAGGACGGCACUGCCGGGGAGCAGCUG CACAAGGCUAUGAAGCGCUAUGCCCUGGUGCCGGCACU AUCGCCUUCACAGAUGCUCACAUCGAGGUGGACAUCACC UAUGCCGAAUACUUUGAGAUGAGCGUGCGGCUGGCAGAA GCCAUGAAGCGGUAUGGGCUCAAUACCAACCACCGGAUU GUGGUGUGCAGCGAGAAUUCUCUGCAGUUCUUUAUGCCA GUGCUGGGCGCCCUGUUCAUCGGGGUGGCCGUGGCCCCU GCCAACGACAUCUACAACGAGCGGGAGCUGCUGAAUUCC AUGGGAAUUUCCCAGCCAACAGUGGUGUUUGUGAGCAAA AAAGGACUGCAGAAGAUUCUCAACGUGCAGAAGAAGCUG CCCAUUAUCCAGAAGAUCAUUAUCAUGGACAGCAAGACU GACUACCAGGGGUUCCAGUCCAUGUAUACCUUUGUGACC AGCCACUUGCCUCCAGGCUUUAACGAAUACGAUUUCGUG CCUGAGUCCUUUGACAGAGACAAGACCAUUGCCUUGAUU AUGAACUCCAGCGGGUCCACAGGAUUGCCCAAGGGGGUG GCCUUGCCCCAUCGGACCGCCUGCGUGAGAUUUCCCACG CCCGCGAUCCCAUCUUCGGCAACCAGAUCAUCCCCGACAC AGCCAUCCUGUCCGUGGUGCCCUUCCACCACGGAUUUGGC AUGUUCACCACCCUGGGCUACUUGAUCUGUGGCUUCAGG GUGGUGUUAAUGUAUCGCUUUGAGGAGGAGCUGUUUCUC CGCAGCCUGCAGGACUACAAGAUCCAGUCUGCUCUGCUG GUGCCCACCCUGUUCAGCUUUUUCGCCAAGAGCACUCUG AUUGACAAGUACGAUCUGAGCAAUCUGCACGAGAUUGCC AGCGGGGGGGCCCCACUGAGCAAGGAGGUCGGGGAGGCU GUGGCCAAGCGGUUCCACCUGCCAGGCAUCCGGCAGGGG UACGGCCUGACCGAGACCACCUCUGCUAUUCUGAUUACCC CCGAGGGAGAUGAUAAGCCUGGGGCUGUGGGGAAGGUGG UGCCCUUCUUUGAAGCCAAGGUGGUGGACCUGGAUACAG GGAAGACCCUGGGGGUGAAUCAGCGCGGGGAGCUCUGUG UGCGCGGCCCCAUGAUCAUGAGCGGCUACGUGAACAACC CAGAAGCUACCAACGCCCUGAUUGACAAAGACGGCUGGC UGCACUCCGGGGACAUCGCCUACUGGGAUGAAGAUGAGC ACUUCUUCAUCGUGGACCGCCUGAAGUCCCUGAUUAAGU AUAAGGGAUAUCAGGUGGCCCCUGCUGAGUUGGAGUCCA UCCUGCUGCAGCACCCCAAUAUUUUCGAUGCUGGAGUGG |
| | | CUGGACUCCCCGACGACGACGCCGGGGAGCUGCCCGCUGC CGUGGUGGUGCUGGAGCACGGAAGACCAUGACCGAGAA GGAGAUCGUGGACUACGUGGCCAGCCAGGUGACCACCGC CAAGAAGCUGCGCGGAGGCGUGGUGUUCGUGGACGAAGU GCCCAAGGGCCUGACAGGAAAGCUGGAUGCCCGGAAGAU UCGGGAGAUCCUGAUUAAAGCCAAGAAGGGCGGCAAGAU UGCCGUGUAAA |
| 47 | HEPO_CD S | AUGGGGGUGCACGAAUGUCCUGCCUGGCUGUGGCUUCUC CUGUCCCUGCUGUCGCUCCCUCUGGGCCUCCCAGUCCUGG GCGCCCCACCACGCCUCAUCUGUGACAGCCGAGUCCUGGA GAGGUACCUCUUGGAGGCCAAGGAGGCCGAGAAUAUCAC GACGGGCUGUGCUGAACACUGCAGCUUGAAUGAGAAUAU CACUGUCCCAGACACCAAAGUUAAUUUCUAUGCCUGGAA GAGGAUGGAGGUCGGGCAGCAGGCCGUAGAAGUCUGGCA GGGCCUGGCCCUGCUGUCGGAAGCUGUCCUGCGGGGCCA GGCCCUGUUGGUCAACUCUUCCCAGCCGUGGGAGCCCCUG CAGCUGCAUGUGGAUAAAGCCGUCAGUGGCCUUCGCAGC CUCACCACUCUGCUUCGGGCUCUGGGAGCCCAGAAGGAA GCCAUCUCCCCUCCAGAUGCGGCCUCAGCUGCUCCACUCC GAACAAUCACUGCUGACACUUUCCGCAAACUCUUCCGAG UCUACUCCAAUUUCCUCCGGGGAAAGCUGAAGCUGUACA CAGGGGAGGCCUGCAGGACAGGGGACAGAUAAA |
| 48 | 3BNT | CUGGUACUGCAUGCACGCAAUGCUAGCUGCCCCUUUCCCG UCCUGGGUACCCCGAGUCUCCCCCGACCUCGGGUCCCAGG UAUGCUCCCACCUCCACCUGCCCCACUCACCACCUCUGCU AGUUCCAGACACCUCCCAAGCACGCAGCAAUGCAGCUCAA AACGCUUAGCCUAGCCACACCCCCACGGGAAACAGCAGUG AUUAACCUUUAGCAAUAAACGAAAGUUUAACUAAGCUAU ACUAACCCCAGGGUUGGUCAAUUUCGUGCCAGCCACACC |
| 49 | 3u27-28 | GAAGUAAGCUCUGCAUGUUCCCCUUCCUGGGAGGAAACC AAUUGUCAGAGGCUGGACAGGUUUGCCAUCAGAGUGGAU AUACCG |
| 50 | 3u35 | GGCUGAACUCGUCCAUGCUGUGGUAAUGCUGGUGGCAGC UGUGCCACUCCC |
| 51 | 3u36 | GGCUGΛΛCUCGUCCΛUGCUGUGGUΛΛUGCUGGUGGCΛGC UGUGCCACUCCCAGGUGUGCCGUGGCCGGUUGGGGA |
| 52 | 3u37 | GCUGGUGGCAGCUGUGCCACUCCCAGGUGUGCCGUGGCC GGUUGGGA |
| 53 | 3u38 | CAGGUGUGCCGUGGCCGGUUGGGGAGGAAGUCUGCUGUG CCCUGG |
| 54 | T7-P promoter | UAAUACGACUCACUAUAAGG |
| 55 | 5'UTR-Kozak | AAUAAACUAGUAUUCUUCUGGUCCCCACAGACUCAGAGA GAACCCGCCACC |

## Claims

1. An artificial nucleic acid molecule comprising:
(a) a coding region of a polypeptide or protein; and
(b) at least one 3'-untranslated region (3'-UTR) element, wherein the 3'-UTR element comprises or consists of:
an RNA sequence as shown in any one of SEQ ID NOs: 1-23 or 50-53, an extended long sequence thereof, or a DNA sequence corresponding thereto.

2. The artificial nucleic acid molecule according to claim 1, wherein the extended long sequence is an RNA sequence formed by multiple identical or different RNA sequences selected from SEQ ID NOs:1-23 and 50-53 in tandem, either directly or through a linker, or its corresponding DNA sequence;
preferably, wherein said multiple refers to 2-10, preferably 2-5, and more preferably 2-3 sequences; preferably, wherein the extended long sequence is selected from:
an RNA sequence as shown in any one of SEQ ID NOs: 24-44 or 49, or an extended long sequence thereof, or a DNA sequence corresponding thereto.

3. The artificial nucleic acid molecule according to claim 1 or 2, wherein the 3'-untranslated region comprises or consists of an RNA sequence as shown in any one of SEQ ID No.3, 8, 10, 13, 14, 24, 26, 27, 29, 33, 36, 38, 40, 44, 49 to 53, an extended long sequence thereof, or a DNA sequence corresponding thereto.

4. The artificial nucleic acid molecule according to any of claims 1 to 3, wherein said artificial nucleic acid molecule further comprises:
(c) a promoter; and/or
(d) at least one 5'-untranslated region elements;
preferably, under the control of said (c) promoter, said (a) coding region and (b) 3'UTR element, and optionally said (d) 5'UTR element, are capable of being transcribed to produce one transcript, wherein the nucleic acid sequence transcribed from said (b) 3'UTR element is functional to enhance the translation efficiency and/or stability of the nucleic acid sequence transcribed from said (a) coding region.

5. The artificial nucleic acid molecule according to claim 4, wherein said artificial nucleic acid molecule further comprises one or more of the following elements: replication origin, enhancer, screening marker and restriction enzyme site.

6. The artificial nucleic acid molecule according to any one of claims 1 to 5, wherein said artificial nucleic acid molecule is a DNA molecule or an RNA molecule;
preferably, the RNA molecule is mRNA, self-replicating RNA, or circular RNA;
when the artificial nucleic acid molecule is an mRNA molecule, the mRNA molecule preferably comprises a polyadenylate tail at the 3' end; preferably, the polyadenylate tail has a length of 20 to 200 nucleotides, more preferably 60 to 150 nucleotides.

7. The artificial nucleic acid molecule according to any one of claims 1 to 6, wherein said coding region encodes at least one polypeptide or protein;
preferably, the polypeptide or protein is a therapeutic and/or prophylactic polypeptide or protein.

8. A vector comprising the artificial nucleic acid molecule according to any of claims 1 to 7.

9. A host cell comprising the vector according to claim 8.

10. A pharmaceutical composition comprising the artificial nucleic acid molecule according to any of claims 1 to 7, or the vector according to claim 8, and/or the host cell according to claim 9, and a pharmaceutically acceptable carrier,
preferably, said pharmaceutical composition is used for DNA or RNA-based therapy.

11. Use of the artificial nucleic acid molecule according to any of claims 1 to 7, or the vector according to claim 8, or the host cell according to claim 9, and/or the pharmaceutical composition according to claim 10 in the preparation of DNA or RNA-based drugs.

12. A kit comprising the artificial nucleic acid molecules according to any of claims 1 to 7, the vector according to claim 8, the host cells according to claim 9, and/or the pharmaceutical composition according to claim 10.

13. Use of one or more 3' non-translational region elements according to claim 1 (b) for translating the coding region of a nucleic acid molecule into a polypeptide or protein encoded by said coding region.

14. Use of one or more 3' non-translational region elements according to claim 1 (b) for translating the coding region of a nucleic acid molecule into a polypeptide or protein encoded by said coding region, preferably, the enhancement in translational performance is an increase in RNA translation efficiency and/or stability; more preferably, the improvement in RNA translation efficiency is reflected in the increased total expression level of the polypeptide or protein.

15. A method for obtaining RNA, wherein said method includes:
(i) providing the artificial nucleic acid molecule according to claim 1 to 7, and
(ii) using the artificial nucleic acid molecule as a template for transcription or replication to obtain RNA.

16. A method for obtaining a polypeptide or protein, wherein said method includes:
(i) obtaining RNA encoding the polypeptide or protein according to claim 14, and
(ii) translating said RNA.
